# NEUE EUROPÄISCHE PATENTSCHRIFT

(11) **EP 0 566 050 B2**
(45) Veröffentlichungstag und Bekanntmachung des Hinweises auf die Entscheidung über den Einspruch: **09.07.2008**
(45) Hinweis auf die Patenterteilung: 03.09.1997
(21) Anmeldenummer: 93105916.6
(22) Anmeldetag: 13.04.1993
(51) Int. Cl.: C12Q 1/68, C12Q 1/70

(54) **Verfahren zum Nachweis von Nukleinsäuren**
Method for the detection of nucleic acids
Procédé pour la détection d'acides nucléiques

(30) Priorität: 15.04.1992 DE 4212555
(43) Veröffentlichungstag der Anmeldung: 20.10.1993
(73) Patentinhaber: Roche Diagnostics GmbH, 68305 Mannheim (DE)
(72) Erfinder: Kruse-Müller, Cornelia, Dr., W-8132 Tutzing (DE); Köhler, Stefanie, Dr., W-8130 Starnberg (DE)
(74) Vertreter: Böhm, Brigitte

(56) Entgegenhaltungen:
- EP-A- 0 261 955
- EP-A- 0 281 927
- EP-A- 0 428 197
- WO-A-89/05357

## Beschreibung

Gegenstand der Erfindung ist ein Verfahren zum Nachweis von Nukleinsäuren, die Verwendung bestimmter Detergentien zur Stabilisierung von einzelsträngigen Nukleinsäuren, sowie ein Reagenzkit und Analysesystem zur Ausführung des Verfahrens.

Auf dem Gebiet der Nukleinsäurehybridisierungstests wurden im letzten Jahrzehnt beträchtliche Fortschritte in Bezug auf Ausnützung der spezifischen Hybridisierung von zueinander komplementären Nukleinsäuren gemacht. So ist es mittlerweile möglich, viele Parameter, z.B. Infektionen, nicht nur durch immunologische Tests, sondern auch durch Nukleinsäurehybridisierungstests nachzuweisen. Besonders förderlich war diesem Ziel die Möglichkeit, Nukleinsäuren spezifisch vor ihrem Nachweis in-vitro zu vermehren und somit auch Nukleinsäuren zu erfassen, welche in nur geringen Mengen in Proben enthalten sind.

Derartige Nukleinsäuretests fanden jedoch bislang wegen ihrer relativ hohen Komplexität nur Verwendung in Einzeltests, welche eine aufwendige von Hand Bearbeitung der Probe erforderten. Ein typisches Beispiel für einen solchen Test ist in der EP-A-0 200 362 beschrieben. In diesem Verfahren werden die amplifizierten Nukleinsäuren einer Auftrennung durch Gelelektrophorese unterzogen. Anschließend wird mit spezifischen markierten Nukleinsäuresonden die Anwesenheit der Nukleinsäuren im Gel sichtbar gemacht. Solche Verfahren sind für Routinediagnostik in Labors mit hohem Probendurchsatz praktisch nicht brauchbar.

Aus der EP-A-0 261 955 ist ein Verfahren zum Nachweis von Bakterien bekannt, welches aus den Schritten Lyse der Zellwände (durch Proteinase K), dann Alkalibehandlung zur Denaturierung der DNS und anschließend Immobilisierung der einzelsträngigen Nukleinsäuren durch Zusatz eines chaotropen Agenzes besteht. Auch dieses Verfahren ist wegen der komplizierten Einzelschritte, insbesondere der direkten Immobilisierung der nachzuweisenden Nukleinsäure auf einer Membran, nicht in Routinelabors durchführbar.

Zur Lyse von Bakterien sind aus Maniatis et al. (Molecular Cloning, Edt. Sambrook et al., Cold Spring Harbour 1989) Bedingungen bekannt, welche SDS (Natriumdodecylsulphat) unter alkalischen Bedingungen beinhalten. Auch dort findet der Nachweis der Nukleinsäuren nach direkter Immobilisierung der Nukleinsäure und Hybridisierung mit einer Detektorsonde auf einer Membran statt. Die Bedingungen sind zwar für molekularbiologische Laboratorien geeignet, nicht jedoch für die Routinediagnostik.

Zur Stabilisierung von DNA wurde bisher in neutraler oder leicht saurer bzw. basischer Lösung ein Puffer verwendet (z. B. TE: 10 mM Tris x HCl, pH 7.4, 1 mM EDTA) oder die DNA in Wasser aufbewahrt oder lyophilisiert bzw. bei -20 °C eingefroren oder als Ethanolpräzipitat bei -20 °C gelagert. Diese Bedingungen sind jedoch ebenfalls praktisch nicht in Routineanalyseverfahren, bei denen es auf hohen Durchsatz und einfache Handhabung ankommt, einsetzbar.

Zur Verringerung unspezifischer Bindungen an Filter wurde der Zusatz von Fremd-DNA (z.B. Heringssperm-DNA) vorgeschlagen. Als Zusätze zu Lösungen, die eine Hybridisierung von Nukleinsäuren erleichtern sollen, wurden Polyvinylpyrrolidon, Rinderserumalbumin, Ficoll^{R} und SDS vorgeschlagen.

In EP-A-0 132 948 ist beschrieben, dass SB-16 die unspezifische Bindung eines Konjugates aus Avidin und Alkalischer Phosphatase an Nitrocellulosefilterplättchen reduziert.

Beschrieben ist ferner die Silikonisierung von Glas- und Kunststoffgefäßen zur Stabilisierung geringer Mengen DNA bzw. Einzelstrang DNA.

In EP-A-0 258 017 ist die Stabilisierung der DNA-Polymerase aus Thermus aquaticus durch nichtionische Detergenzien beschrieben.

Sanchez-Pescador et al. (Antimicrobiol Agents and Chemotherapy, vol. 33, No. 10 (1989) 1813-1815) beschreiben einen Nukleinsäureassay zum Nachweis von Bakterien mit tetM-vermittelter Tetracyclinresistenz. Dabei werden Zellen einer Lysebehandlung unterzogen, wobei die Lyselösung u.a. Proteinase K und SDS enthält. Es wird dann eine Lösung zugegeben, die neben Fangsonden auch NaOH enthält. Eine stabilisierende Wirkung von SDS auf einzelsträngige Nukleinsäuren in alkalischen Lösungen wird bei Sanchez-Pescador et al. nicht erwähnt.

Urdea et al. ("Luminescence Immunoassay and Molecular Applications", Bocaraton, Florida, CRC Press (1990)) beschreiben ein Verfahren zur Detektierung von Hepatitis B Virus. Dazu wird zunächst eine Lyse mit einer Lyselösung durchgeführt, welche Proteinase H und SDS enthält. Die freigesetzte DNA wird dann zur weiteren Analyse herangezogen, wobei der DNA-haltigen Lyselösung dann Fangsonden und Natriumhydroxyd gleichzeitig zugegeben werden. Die Möglichkeit, einzelsträngige Nukleinsäuren durch Detergentien zu stabilisieren, wird bei Urdea et al. nicht erwähnt.

WO 91/19567 beschreibt u.a. die Automatisierung von DNS-Analyse-Assays. WO 91/19567 beschreibt aber nicht Reagenzaufbewahrungsgefäße mit einer Denaturierungslösung, die ein anionisches, nichtionisches oder zwitterionisches Detergenz enthält.

EP 0 317 077 A1 beschreibt Kits für Hybridisierungsassays für amplifizierte Nukleinsäuren, die typischerweise in getrennten Behältern u.a. eine Fangsonde, eine zur Immobilisierung des Analyts bestimmte Festphase und ein Denaturierungsreagenz enthalten. Nicht beschrieben wird, dass die Analkalihydroxid-Lösung ein anionisches, nichtionisches oder zwitterionisches Detergenz enthalten sollte.

Aufgabe der vorliegenden Erfindung war es, ein Verfahren zum Nachweis von Analytnukleinsäuren bereitzustellen, welches den Bedürfnissen von Routinediagnostiklabors besser gerecht wird.

Gegenstand der Erfindung ist ein Verfahren zum Nachweis einer Nukleinsäure enthaltend nacheinander folgende Schritte in der Reihenfolge:
Einzelsträngigmachen der Nukleinsäure unter alkalischen Bedingungen;
Zugabe einer immobilisierbaren oder immobilisierten Fangsonde;
Hybridisierung der Fangsonde mit der Nukleinsäure unter Immobilisierung der Nukleinsäure über die Fangsonde; und
Nachweis der Menge an gebildetem immobilisiertem Hybrid,
wobei während der Behandlung unter alkalischen Bedingungen mindestens ein Detergenz aus der Gruppe der anionischen, nichtionischen und zwitterionischen Detergentien vorhanden ist, wobei das Einzelsträngigmachen in einem Gefäß aus Polystyrol, Polyethylen oder Luran vorgenommen wird.

Ebenfalls Gegenstand der Erfindung ist die Verwendung bestimmter Detergentien zur Stabilisierung von Nukleinsäuren in alkalischen Lösungen sowie ein Reagenzkit und Analysesystem zur Durchführung des Verfahrens.

Bei dem erfindungsgemäßen Verfahren handelt es sich um eine spezielle Ausführungsform der sogenannten Hybridisierungstests, die in ihren Grundzügen dem Fachmann auf dem Gebiet der Nukleinsäurediagnostik bekannt sind. Soweit experimentelle Details im folgenden nicht ausgeführt sind, wird dazu vollinhaltlich auf "Nucleic acid hybridisation", Herausgeber B.D. Hames und S.J. Higgins, IRL Press, 1986, z.B. in den Kapiteln 1 (Hybridisation Strategy), 3 (Quantitative Analysis of Solution Hybridisation) und 4 (Quantitative Filter Hybridisation), Current Protocols in Molecular Biology, Edt. F.M. Ausubel et al., J. Wiley and Son, 1987, und Molecular Cloning, Edt. J. Sambrook et al., CSH, 1989, Bezug genommen. Zu den bekannten Methoden gehört auch die Herstellung von markierten Nukleosidtriphosphaten, wie sie in EP-A-0 324 474 beschrieben sind, die chemische Synthese von modifizierten und unmodifizierten Oligonukleotiden, die Spaltung von Nukleinsäuren mittels Restriktionsenzymen, die Auswahl von Hybridisierungsbedingungen, durch welche eine Spezifität erreicht werden kann, die vom Ausmaß der Homologie zwischen den zu hybridisierenden Nukleinsäuren, deren GC-Gehalt und deren Länge abhängt, sowie die Bildung von Nukleinsäuren aus Nukleosidtriphosphaten mit Hilfe von Polymerasen, gegebenenfalls unter Verwendung von sogenannten Primern.

Eine Markierung im Sinne der vorliegenden Erfindung besteht aus einer direkt oder indirekt nachweisbaren Gruppe L. Direkt nachweisbare Gruppen sind beispielsweise radioaktive (³²P), farbige, oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da an mit ihnen markierte Nukleosidtriphosphate im allgemeinen besonders gut als Substrate von Polymerasen einsetzbar sind und eine anschließende Reaktion mit einem markierten Antikörper gegen das Hapten oder das haptenisierte Nukleosid leicht vorgenommen werden kann. Solche Nukleosidtriphosphate sind beispielsweise Brom-Nukleosidtriphosphate oder Digoxigenin-, Digoxin- oder Fluorescein-gekoppelte Nukleosidtriphosphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

Nukleosidtriphosphate (NTP) sind Ribo (rNTP)- oder Desoxyribonukleosidtriphosphate (dNTP).

Unter einer Analytnukleinsäure wird eine Nukleinsäure verstanden, die Ziel des Nachweises ist. Unter Analytnukleinsäuren sind hierbei Nukleinsäuren jeglichen Ursprungs zu verstehen, beispielsweise Nukleinsäuren viroiden, viralen, bakteriellen oder zellulären Ursprungs. Sie können in Lösung, Suspension, aber auch an Festkörpern fixiert oder in zellhaltigen Medien, Zellabstrichen, fixierten Zellen, Gewebsschnitten oder fixierten Organismen vorliegen. Bevorzugt liegen die Nukleinsäuren in Lösung vor. Die Reaktionssequenz wird meist gestartet durch Verfügbarmachung der Analytnukleinsäure mit entsprechenden Reagentien. Hierbei können sowohl Veränderungen des pHs (alkalisch), Hitze, Wiederholung extremer Temperaturveränderungen (Einfrieren/Auftauen), Veränderung der physiologischen Wachstumsbedingungen (Osmotischer Druck), Einwirkung von Detergentien, chaotropen Salzen oder Enzymen (z.B. Proteasen, Lipasen), alleine oder in Kombination zur Freisetzung der Nukleinsäuren beitragen. Da das erfindungsgemäße Verfahren sehr empfindlich und selektiv ist, können auch kleine Nukleinsäuremengen in Anwesenheit anderer Stoffe, beispielsweise Proteinen, Zellen, Zellfragmenten, aber auch nicht nachzuweisender Nukleinsäuren nachgewiesen werden. Eine Aufreinigung von Proben kann somit entfallen, wenn sichergestellt ist, daß die nachzuweisenden Nukleinsäuren für die eingesetzten Reagenzien ausreichend zugänglich sind.

Eine template-Nukleinsäure ist eine Nukleinsäure, zu der ein im wesentlichen komplementärer Nukleinsäurestrang neu gebildet wird. In Bezug auf die Sequenzinformation dient die template-Nukleinsäure als Matritze für die Umschreibung.

Denaturierung von Nukleinsäuren bedeutet Auftrennung von Nukleinsäuredoppelsträngen in Einzelstränge. Dem Fachmann stehen prinzipiell eine Vielzahl von Möglichkeiten zur Verfügung, z.B. Behandlung durch Alkalihydroxide, durch Hitze, oder durch Chemikalien.

Unter einem spezifischen Nachweis wird ein Verfahren verstanden, durch welches gewünschtenfalls selektiv bestimmte Nukleinsäuren auch in Gegenwart anderer Nukleinsäuren nachgewiesen werden können. Es ist jedoch auch möglich, eine Gruppe Nukleinsäuren mit teilweise übereinstimmender oder ähnlicher Nukleotidsequenz nachzuweisen. Zum Nachweis doppelsträngiger Nukleinsäuren kann jeder der beiden komplementären Stränge einbezogen werden.

Unter einer zu einer Nukleinsäure im wesentlichen komplementären Nukleinsäure oder Nukleinsäuresequenz werden Nukleinsäuren oder Sequenzen verstanden, die mit der entsprechenden Nukleinsäure hybridisieren können, deren Nukleotidsequenz im hybridisierenden Bereich entweder genau komplementär zu der anderen Nukleinsäure ist oder sich in wenigen Basen von der genau komplementären Nukleinsäure unterscheidet. Die Spezifität richtet sich dabei sowohl nach dem Grad der Komplementarität als auch nach den Hybridisierungsbedingungen.

Flüssige Phasen sind die üblicherweise bei Nukleinsäurenachweisen verwendeten wässrigen Phasen, gegebenenfalls mit gelösten organischen bzw. anorganischen Inhaltsstoffen, z.B. Puffersubstanzen, überschüssigen Reagenzien, nicht nachzuweisenden Nukleinsäuren, Proteinen etc..

Die nachzuweisende Nukleinsäure kann die Analytnukleinsäure selbst sein. Sie kann aber auch eine Nukleinsäure sein, die aus der Analytnukleinsäure durch Vorbehandlung in der Probenflüssigkeit hergestellt wurde. Zu den bekannten Vorbehandlungen gehört insbesondere die Fragmentierung, beispielsweise mittels Restriktionsenzymen, oder die cDNS-Synthese aus RNS. Damit das erfindungsgemäße Verfahren seine Vorteile voll entfalten kann, hat es sich als zweckmäßig erwiesen, wenn die nachzuweisende Nukleinsäure eine Größe von mindestens 40 bp aufweist.

Bevorzugt ist die nachzuweisende Nukleinsäure das Produkt einer vorgeschalteten spezifischen oder unspezifischen Nukleinsäurevermehrung. Solche Nukleinsäureamplifikationsverfahren sind beispielsweise aus EP-A-0 201 184, EP-A-0 237 362, EP-A-0 329 822, EP-A-0 320 308 oder WO 88/10315 bekannt. In diesen Verfahren dient die Analytnukleinsäure als template-Nukleinsäure für die Herstellung der letztendlich nachzuweisenden Nukleinsäure.

Die Nukleinsäure kann jedoch auch eine durch Klonierung und in-vivo-Vermehrung hergestellte Nukleinsäure sein.

Beispielsweise kann ein Verfahren zum Nachweis eines speziellen Virus in einer Körperflüssigkeit (z.B. Serum) als ersten Schritt die Lyse der Virushülle enthalten. Verfahren zur Lyse von Zellwänden sind dem Fachmann bekannt. Beispielsweise kann die Lyse durch Behandlung mit Alkalihydroxydlösungen vorgenommen werden. Auch der Zusatz von Hilfsstoffen, z.B. Detergentien, ist möglich. Daran kann sich insbesondere bei Viren, welche nur in geringen Konzentrationen in Körperflüssigkeiten vorliegen (z.B. Hepatitis C-Virus), eine in vitro Nukleinsäurevermehrung anschliessen (z.B. über die Polymerase Chain Reaction oder die anderen oben genannten Amplifikationsverfahren). Dabei wird mit der Analytnukleinsäure als Templatnukleinsäure eine Vielzahl von Nukleinsäuren gebildet, welche dann in dem erfindungsgemäßen Verfahren nachgewiesen werden.

Bei einem Nachweis von Bakterien, beispielsweise in Lebensmitteln, könnten dem erfindungsgemäßen Verfahren ebenfalls mehrere Schritte vorgeschaltet werden. Im allgemeinen werden Bakterienproben ggf. nach in vivo Vermehrung der Bakterien unter Bedingungen, welche die Lyse der Bakterienzellwand bewirken (z.B. Proteinasen, Alkali) aufgeschlossen. Bei sehr niedrig konzentrierten Proben kann sich auch hier eine in vitro Amplifikation der Analytnukleinsäuren des Bakteriums anschließen.

Resultat der diversen Vorbehandlungen ist immer eine Probenflüssigkeit, welche Nukleinsäuren gelöst enthält und in der ggf. die in den vorbereitenden Schritten eingesetzten Reagenzien sowie gegebenenfalls zerstörte Zellbestandteile enthalten sind.

Das erfindungsgemäße Verfahren sieht nun die Behandlung der Probe unter alkalischen Bedingungen vor, wobei auch mindestens ein Detergenz aus der Gruppe der anionischen, nichtionischen und zwitterionischen Detergentien vorhanden ist. Diese Bedingungen sind darauf abgestellt, die in der Flüssigkeit vorhandenen Nukleinsäuren einzelsträngig zu machen, sofern sie es noch nicht sind. Dazu wird der Flüssigkeit ein Alkalihydroxyd, bevorzugt Natriumhydroxyd, in Form eines Feststoffes oder bevorzugt als Lösung (Denaturierungslösung) in einer Menge zugegeben, dass der pH der Probenflüssigkeit nach der Zugabe zwischen 9,5 und 14, bevorzugt 11 und 13,5, liegt. Die zugegebene Lösung kann auch noch weitere Hilfsstoffe (z.B. Salze und Puffer) enthalten.

Kern der Erfindung ist die Erkenntnis, dass die Anwesenheit bestimmter Detergentien während der Behandlung unter alkalischen Bedingungen zur Einzelsträngigmachung erhebliche Vorteile mit sich bringt. Zur Ausführung der Erfindung wird dieses Detergenz der Probenflüssigkeit entweder in Festform oder bevorzugt in Form einer Lösung zugegeben, sodass die Konzentration des Detergenz in der Probenflüssigkeit 0,01 Gew.% bis 10 Gew.%, bevorzugt 0,05 Gew.% bis 5 Gew. %, beträgt. Es ist möglich, das Detergenz zu der vorbereiteten Probenflüssigkeit schon vor, aber auch nach Einstellung der alkalischen Bedingungen zuzugeben. Besonders bevorzugt wird das Detergenz in oder zusammen mit der alkalischen Denaturierungslösung zu der Probenflüssigkeit zugegeben. Die Konzentration an Detergenz in der Denaturierungslösung beträgt bevorzugt 0.01 bis 15 Gew. %, besonders bevorzugt 0.05 bis 10 Gew. %.

Bevorzugte nichtionische Detergentien sind Synperonic (Blockcopolymer aus Poloxyethylen und Polyethoxypropylen, Pharmacia), Tween 20 (Polyethylenglykol (20) Sorbitanmonolaurat), Thesit^{R} (Dodecylpolyethylenglycolether), Triton X-100 (Polyethylenglykol (9-10) p-t-Oktylphenol), NP-40 (Ethylenphenolpolyethylenglycolether) und glykosidische Detergentien, z.B. Octyl- beta -D-glukopyranosid. Besonders wirksam im Sinne der Erfindung sind Synperonic und NP-40.

Beispiele für anionische Detergentien sind SDS (Natriumlaurylsulphat), Natriumlaurylsarcosin und Natriumdesoxycholat. Besonders bevorzugt aus dieser Gruppe ist SDS.

Bei den zwitterionischen Detergentien sind Zwittergent 3-08 (N-Oktyl-N,N-dimethyl-3-ammonio-1-propansulfonat), Zwittergent 3-12 (N-Docecanyl-N,N-dimethyl-3-ammonio-1-propansulfonat), CHAPS (3-[(3-Cholamidopropyl)-dimethylammonio]-1-propansulfonat) und CHAPSO (3-[(3-Colamidopropyl)-dimethylamino]-2-hydroxy-1-propansulfonat) bevorzugt. Als besonders wirksam hat sich Zwittergent 3-12 herausgestellt.

Die Bedingungen für das Einzelsträngigmachen können gemäß der vorliegenden Erfindung zwischen 1 Min. und 8 Std. aufrechterhalten werden, ohne dass eine merkliche Zersetzung der nachzuweisenden Nukleinsäuren stattfindet. Bevorzugt sind nach 4 Stunden noch mehr als 90 % der ursprünglich vorhandenen Nukleinsäuren nachweisbar.

Bevorzugt findet die Denaturierung in einem Kunststoffgefäß statt. Insbesondere sind dabei solche Gefäße einsetzbar, welche normalerweise in üblichen Analysenautomaten als Probenaufbewahrungsgefäße verwendet werden. Die Gefäße sind aus Polystyrol, Polyethylen oder Luran. Während der Behandlung mit Alkali und Detergenz befindet sich das Gefäß, in dem die Behandlung stattfindet, bevorzugt schon in einem Analysenautomaten, besonders bevorzugt auf einem Probenrotor, welcher den Zugriff einer Probenentnahmevorrichtung auf einzelne auf dem Rotor befindliche Probengefäße zulässt. Ein solcher Analysenautomat ist beispielsweise beschrieben in EP-A- 0 361 830.

Besonders positiv wirkt sich die Erfindung bei Analysenautomaten und Analysenverfahren aus, bei denen die Standzeiten der gefüllten Probenaufbewahrungsgefäße von Gefäß zu Gefäß variieren und somit die Zeiten der Behandlung mit Alkali systembedingt unterschiedlich lang sind. Diese Automaten haben haben den Vorteil, daß im Notfall die Analyse bestimmter Proben vorgezogen werden kann, was die Analyse anderer Proben auf einen späteren Zeitpunkt verschiebt.

Ein weiterer wesentlicher Schritt des erfindungsgemäßen Verfahrens ist die Zugabe einer immobilisierbaren oder immobilisierten Fangsonde. Dies kann geschehen, indem eine Lösung einer immobilisierbaren Fangsonde zu der Lösung mit der einzelsträngigen Nukleinsäure zugegeben, beispielsweise zupipettiert, wird, bzw. mit einem Feststoff, an welchen eine Fangsonde gebunden ist in Kontakt gebracht wird.

Unter einer immobilisierbaren Fangsonde wird eine Nukleinsäure verstanden, welche strukturell gegenüber normalen, zu der nachzuweisenden Nukleinsäure im wesentlichen komplementären Nukleinsäure dahingehend verändert ist, daß sie eine oder mehrere zur Immobilisierung befähigende Gruppen I aufweisen. Bevorzugt ist der Fall, daß es sich bei der Fangsonde um eine einzelsträngige Nukleinsäuresonde handelt und der dazu komplementäre Strang nicht zugesetzt wird. Die Lösung mit der einzelsträngigen Fangsonde enthält außerdem bevorzugt zur Hybridisierung hilfreiche Reagenzien, wie z.B. SSC, Formamid (bei längeren Fragmenten) oder Blockierungsreagenzien für nicht nachzuweisende Nukleinsäuren sowie bevorzugt Reagenzien zur korrekten Einstellung des pH-Wertes. Besonders bevorzugt ist die Fangsonde eine Nukleinsäure gemäß DE-A-41 23 540.

Einzelsträngige Fangsonden können beispielsweise durch chemische Nukleinsäuresynthese gemäß DE-A- 39 16 871 oder auch gemäß EP-B-0 184 056 hergestellt werden.

Bei der Durchführung des Nachweisverfahren auf einem automatischen Analyzer wird die Hybridisierungslösung bevorzugt automatisch aus einem Reagenzaufbewahrungsgefäß, welches beispielsweise auf einem Rotor, der Reagenzien für mehrere, unterschiedliche Bestimmungen enthält, aufgenommen und mit einem Aliquot der Flüssigkeit mit der denaturierten nachzuweisenden Nukleinsäure vereinigt. Besonders bevorzugt entnimmt der Analyzer zunächst ein bestimmtes Volumen aus einem Probenaufbewahrungsgefäß und dann ein bestimmtes Volumen an Hybridisierungslösung aus einem bestimmten Reagenzaufbewahrungsgefäß und stößt diese in ein Immobilisierungs- und Nachweisgefäß aus.

Zur Immobilisierung befähigende Gruppen I sind beispielsweise chemische Gruppen, die normalerweise in natürlichen Nukleinsäuren nicht vorhanden sind und die kovalent, beispielsweise über eine chemische oder eine Photoreaktion an eine feste Phase gebunden werden können, oder Gruppen bzw. Molekülteile, die über gruppenspezifische Wechselwirkungen von einem anderen Molekül oder Molekülteil erkannt und gebunden werden können. Solche Gruppen sind daher z. B. Haptene, Antigene und Antikörper, Nukleotidsequenzen, Rezeptoren, Regulationssequenzen, Glykoproteine, beispielsweise Lectine, oder auch die Bindungspartner von Bindeproteinen, wie Biotin oder Iminobiotin. Bevorzugt sind Vitamine und Haptene, besonders bevorzugt sind Biotin, Fluorescein oder Steroide, wie Digoxigenin oder Digoxin.

Während der Inkubation der Fangsonde mit der nachzuweisenden einzelsträngigen Nukleinsäure hybridisieren diese miteinander unter Bildung eines Hybrids D. Sofern die Hybridisierung weiterer Nukleinsäuren, z.B. Nachweissonden, mit einzelsträngigen Teilen des Hybrids D beabsichtigt ist, können diese schon in diese Mischung eingebracht werden und wie gewünscht zur Hybridisierung gebracht werden.

Die Flüssigkeit, welche das Nukleinsäurehybrid D gelöst enthält, wenn die nachzuweisende Nukleinsäure in der Probe vorhanden war, wird mit einer festen Phase in Kontakt gebracht, welche das Hybrid D über die immobilisierbaren Gruppen der Nukleinsäuresonde spezifisch binden kann.

Die Art der Festphase richtet sich nach der zur Immobilisierung befähigenden Gruppe I. Bevorzugt weist sie eine immobilisierende Gruppe R auf, die eine bindende Wechselwirkung mit I eingehen kann. Ist die immobilisierbare Gruppe beispielsweise ein Hapten, dann kann eine Festphase verwendet werden, die an ihrer Oberfläche Antikörper gegen dieses Hapten aufweist. Ist die immobilisierbare Gruppe ein Vitamin, wie z. B. Biotin, dann kann die Festphase bindende Proteine, wie Avidin oder Streptavidin immobilisiert enthalten. Besonders bevorzugte Reste I und R sind Biotin und Streptavidin. Die Immobilisierung über eine Gruppe an der modifizierten Nukleinsäure ist besonders vorteilhaft, da sie unter milderen Bedingungen stattfinden kann als beispielsweise Hybridisierungsreaktionen.

Bevorzugt wird zur Immobilisierung der gebildeten Nukleinsäuren die Reaktionsmischung vor, während oder nach Bildung der Nukleinsäurehybride D in ein Gefäß gefüllt, welches an seiner Oberfläche mit der immobilisierbaren Gruppe reagieren kann. Bevorzugt findet die Hybridisierungsreaktion mit der Sonde im wesentlichen gleichzeitig mit der Immobilisierung statt. Es ist möglich, eine Festphase in Form eines porösen Materials, wie einer Membran, eines Gewebes oder eines Vlieses, zu verwenden, auf welche die Reaktionsmischung aufgegeben wird. Ebenso ist die Verwendung von Perlen, sogenannten beads, oder Latex-Partikeln möglich. Das Gefäß ist bevorzugt eine Küvette, ein Röhrchen oder eine Mikrotiterplatte. Die feste Phase sollte mindestens soviele Bindungsstellen für die immobilisierbare Gruppe der Sonde haben, wie Nukleinsäurehybride D und damit nachzuweisende Nukleinsäuren vorhanden sind.

Die Herstellung einer bevorzugten festen Phase ist in der EP-A- 0 344 578 beschrieben, auf welche vollinhaltlich Bezug genommen wird.

In einer besonders bevorzugten Ausführungsform, die analyzergängig ist, wird die Lösung mit der erfindungsgemäß denaturierten Nukleinsäure in ein Gefäß, welches einen spezifischen Bindungspartner für eine immobilisierende Gruppe der Fangsonde an der Oberfläche gebunden enthält, gefüllt. In das Gefäß wird auch eine Hybridisierungslösung zugegeben, welche eine einzelsträngige Fangsonde enthält, die eine immobilisierende Gruppe aufweist. Dadurch wird die Reaktionsmischung neutralisiert. Eventuell anwesende nachzuweisende Nukleinsäure, welche mit der Fangsonde hybridisiert hat, wird an die Festphase gebunden.

Unter einer immobilisierten Fangsonde wird eine zu mindestens einem Teil der nachzuweisenden Nukleinsäure im wesentlichen komplementäre Nukleinsäure verstanden, welche im Moment der Zugabe bereits an eine Festphase gebunden ist. Die Bindung kann kovalent sein, aber auch auf biospezifischen Wechselwirkungen, wie bei den immobilisierbaren Sonden beschrieben, beruhen. Die Festphase kann ein Gefäß sein, aber auch ein Feststoff anderer Geometrie, z.B. eine Kugel, die ein weiteres Gefäß erforderlich macht.

Nach einer Inkubationszeit, während der die Immobilisierungsreaktion stattfindet, wird die flüssige Phase aus dem Gefäß, dem porösen Material oder den pelletierten beads entfernt. Die Festphase kann anschließend mit einem geeigneten Puffer gewaschen werden, da die Bindung der Hybride D an der Festphase sehr effizient ist.

Die Menge des an die Festphase gebundenen Hybrids aus nachzuweisender Nukleinsäure und Fangsonde kann auf im Prinzip bekannte Weise bestimmt werden. Einerseits kann dies unter Verwendung einer Nachweissonde nach Art eines Sandwich-Hybridisierungsverfahrens geschehen. Bei diesem Verfahren wird das Hybrid mit einer Nachweissonde umgesetzt, welche zu einer anderen Nukleotidsequenz der nachzuweisenden Nukleinsäure als die Fangsonde komplementär ist, und mit dieser hybridisiert, umgesetzt. Dadurch bildet sich ein Hybrid aus Nachweissonde, nachzuweisender Nukleinsäure und Fangsonde an der Festphase. Ein solches Verfahren ist beispielsweise beschrieben in der EP-A-0 079 139. Bevorzugt wird die Nachweissonde zusammen mit der Fangsonde in der Hybridisierungslösung zugegeben.

Für den Fall, daß dem erfindungsgemäßen Nachweisverfahren eine Amplifikationsreaktion vorgeschaltet war, ist der Fall bevorzugt, daß während der Amplifikationsreaktion in das Produkt der Vermehrung (welche die nachzuweisende Nukleinsäure darstellt) eine nachweisbare Gruppe eingebaut wurde. Ein solches Verfahren ist beispielsweise beschrieben in der DE-A-4 041 608. Dabei werden der Amplifikationsreaktion markierte Mononukleosidtriphosphate eingebaut. Für diesen Fall entfällt eine Hybridisierung mit einer Nachweissonde, da die nachweisbare Nukleinsäure nachweisbar markiert ist.

Bei direkt nachweisbaren Gruppen, beispielsweise Fluoreszenslabeln, wird die Menge an Markierung fluorometrisch bestimmt. Ist die nachweisbare Gruppe indirekt nachweisbar z.B. ein Hapten, so wird die modifizierte Nukleinsäure bevorzugt mit einem markierten Antikörper gegen das Hapten umgesetzt, wie analog in der EP-A-0 324 474 beschrieben. Die Markierung am Antikörper kann beispielsweise eine Farb- oder Fluoreszenzmarkierung oder bevorzugt eine Enzymmarkierung, wie β-Galactosidase, alkalische Phosphatase oder Peroxidase, sein. Im Falle der Enzymmarkierung wird die Menge an Nukleinsäure über die meist photometrische chemoluminometrische oder fluorometrische Verfolgung einer Reaktion des Enzyms mit einem chromogenen, chemoluminogenen oder fluorogenen Substrat gemessen. Das Meßsignal ist ein Maß für die Menge an ursprünglich vorhandener nachzuweisender Nukleinsäure und somit ggf. an nachzuweisenden Organismen.

Bevorzugt wird auch der Nachweis des gebildeten Hybrids mit Hilfe des Analysenautomats vorgenommen. Dazu wird nach der Inkubation der einzelsträngigen Nukleinsäure mit der Fangsonde und ggf. der Nachweissonde die Probenflüssigkeit von den immobilisierten Hybriden entfernt. Dies kann beispielsweise dadurch geschehen, daß die Flüssigkeit von den immobilisierten Hybriden entfernt wird. Dies hängt von der Art der Festphase ab. Beispielsweise wird bei Verwendung eines Gefäßes die Flüssigkeit aus dem Gefäß entfernt, beispielsweise abpipettiert. Nach einem oder mehreren Waschschritten zur vollständigen Entfernung nicht immobilisierter Fangsonde und ggf. Nachweissonde und nachweisbar markiertem Mononukleosidtriphosphat wird je nach Markierung eine Messung (Direktmarkierung) oder eine Zugabe eines Enzymsubstrats und Messung des freigesetzten Spaltprodukts (Enzymmarkierung) vorgenommen. Dabei dient bevorzugt das während der Hybridisierungsreaktion benutzte Gefäß auch zur Durchführung der Messung.

Dem oben geschilderten kann entnommen werden, daß das erfindungsgemäße Verfahren von der Stufe, bei der die Analytnukleinsäuren gelöst in der vorbereiteten Probenlösung vorliegen, bis zu dem Zeitpunkt, an dem immobilisiertes Hybrid aus nachzuweisender Nukleinsäure und Fangsonde vorliegt, ohne zwischenzeitliche Abtrennung der nachzuweisenden Nukleinsäuren durchgeführt werden kann. Dies bedeutet einen beträchtlichen Handling-Vorteil. Das Verfahren hat außerdem den Vorteil, daß es auf automatischen Analyzern, wie sie für Immuntests (siehe beispielsweise EP-A-098 590) allgemein üblich sind, durchgeführt werden kann. Dies macht auch die simultane Bearbeitung sowohl immunologischer als auch nukleinsäurediagnostischer Parameter auf einem Gerät möglich. Außerdem hat das erfindungsgemäße Verfahren den Vorteil, daß es sehr genau ist. Gegenüber Verfahren, welche ohne Zusatz der bestimmten Detergentien durchgeführt werden, sind die Variationen der Meßergebnisse verschiedener Messungen der gleichen Probe sehr stark unterschiedlich, während sie beim erfindungsgemäßen Verfahren weniger als 5 % betragen. Dies jedoch ist eine der Voraussetzungen für die Durchführung von verlässlichen quantitativen automatisierten Nukleinsäure renachweisen. Insbesondere die Tatsache, dass bei Analyzem die Standzeiten von Probengefäßen sehr stark variierten, ließ eine Durchführung von quantitativen Nukleinsäurehybridisierungstests auf Analyzern für immunologische Tests nicht zu.

Ebenfalls Gegenstand der Erfindung ist ein Reagenzkit zur Immobilisierung einer Nukleinsäure, welches in getrennten Behältern ein für eine zu immobilisierende Nukleinsäure spezifische Fangsonde und ein Reagenz enthaltend ein Alkalihydroxyd und mindestens ein Detergenz aus der Gruppe der anionischen, nichtionischen und zwitterionischen Detergentien und ein Gefäß aus Polystyrol, Polyethylen oder Luran enthält. Das Reagenzkit kann ferner weitere Bestandteile enthalten, die zur Immobilisierung der Nukleinsäure hilfreich sind, beispielsweise ein Kunststoffgefäß, an dem die Nukleinsäure über die Fangsonde immobilisiert werden soll.

Ebenfalls Gegenstand der Erfindung ist ein Reagenzkit zum Nachweis einer Nukleinsäure, welches die Komponenten des Reagenzkits zur Immobilisierung einer Nukleinsäure enthält, sowie mindestens eine weitere Nukleinsäure, die für die zu immobilisierende Nukleinsäure bevorzugt an einer anderen Sequenz als die Fangsonde spezifisch ist. Diese weitere Nukleinsäure ist bevorzugt nachweisbar markiert und stellt eine Nachweissonde dar.

Ebenfalls Gegenstand der Erfindung ist ein Analysesystem zur automatischen Bestimmung von diagnostischen Parametern in Probenlösungen, welches mindestens ein Probenaufbewahrungsgefäß aus Polystyrol, Polyethylen oder Luran in einem Probenrotor, mindestens ein Reagenzaufbewahrungsgefäß auf einem Reagenzrotor enthaltend eine Denaturierungslösung für Nukleinsäuren unter alkalischen Bedingungen mit mindestens einem Detergenz aus der Gruppe der anionischen, nichtionischen und zwitterionischen Detergentien, sowie mindestens ein Reagenzaufbewahrungsgefäß enthaltend eine immobilisierbare oder immobilisierte Parameterspezifische Fangsonde enthält. Dieses Analysesystem enthält ferner ein Gerät zur automatischen Abarbeitung von Nachweisen unter Zugriff auf die Probe, eine Denaturierungslösung und die Fangsonde.

Ebenfalls Gegenstand der Erfindung ist die Verwendung von glykosidischen Detergentien in Verfahren zum Nachweis von Nukleinsäuren. Unter glykosidischen Detergentien werden Verbindungen verstanden, welche Zuckerreste enthalten und die Oberflächenspannung von Flüssigkeiten herabsetzen.

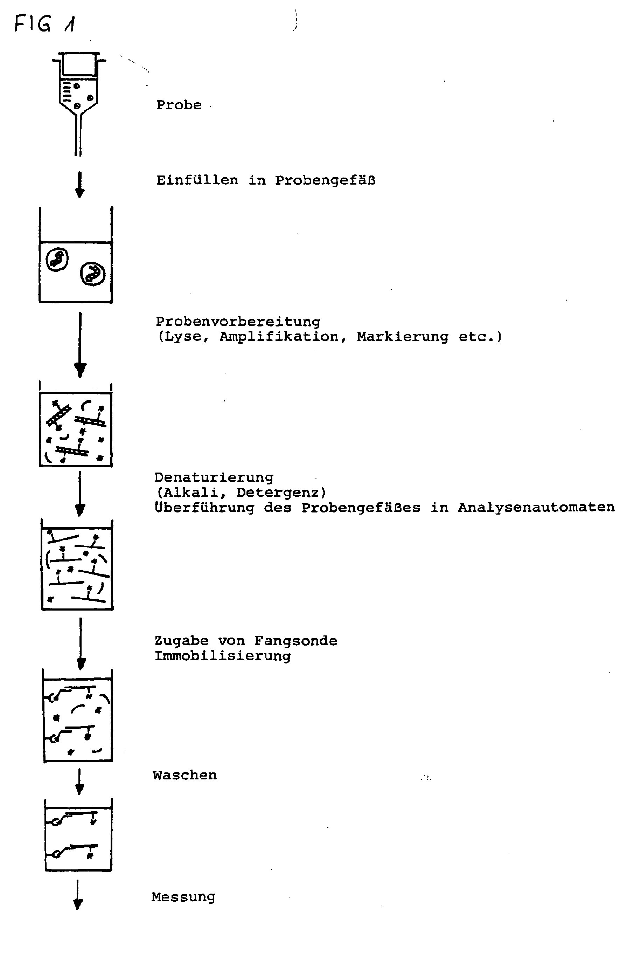

In Fig. 1 ist eine besonders bevorzugte Ausführungsform des erfindungsgemäßen Verfahrens schematisch dargestellt.

In Fig. 2 ist die Stabilität von DNA unter alkalischen Bedingungen graphisch wiedergegeben (Extinktion bei 422 nm). Es ist ersichtlich, dass bei Verwendung von NP-40 die Extinktion der DNA und damit ihre Stabilität nach 60 min fast gleich wie bei Beginn des Experiments ist. Ohne Detergenzzusatz ist die Menge an DNA nach 60 min schon um mehr als die Hälfte gesunken. Dies gilt bei allen drei Konzentrationen von HBV-DNA (40, 80, 200 ng HBV-DNA/ml).

Die nachfolgenden Beispiele erläutern die Erfindung näher.

### Beispiel 1

### Aufschluss von HBV-DNA-haltigen Sera (Lyse)

10 µl HBV-haltiges Serum werden mit 10 µl 0.2 N NaOH gemischt und 1 h bei 37 °C inkubiert und danach mit 30 µl Neutralisationslösung (100 mM KCI, 20 mM Tris/HCl, 3 mM MgCl₂, 0,067 N HCl) versetzt.

Ein Aliquot dieser Mischung wird in die PCR-Amplifikation eingesetzt.

### Beispiel 2

### PCR-Amplifikation und Markierung von HBV-DNA mit Digoxigenin.

20 µl von wie in Beispiel 1 aufgeschlossenen Sera werden nach folgendem Schema in die PCR-Amplifikation eingesetzt:

| Reagenz oder Probe | Konzentration der Vorratslösung | Endkonzentration in in der PCR |
|---|---|---|
| Probe | | 20 µl/100µl Ansatz |
| Primer 1 | 5 µM | 200 nM |
| Primer 2 | 5 µM | 200 nM |
| dATP (Na-Salz) | 4 mM | 200 µM |
| dCTP (Na-Salz) | 4 mM | 200 µM |
| dGTP (Na-Salz) | 4 mM | 200 µM |
| dTTP (Na-Salz) | 4 mM | 175 µM |
| Dig-11-dUTP | 1 mM | 25 µM |
| PCR-Puffer | 10 x | 1 x |
| Taq-Polymerase | 5 U/µl | 2,5 U |

- 10 x PCR Puffer:: 100 mM Tris/HCl, 500 mM KCl, 15 mM MgCl₂, 1 mg/ml Gelatine, pH 9,0
- Dig-11-dUTP:: Digoxigenin-11-dUTP, Li-Salz (Boehringer Mannheim)
- Primer 1:: Oligonukleotid der HBV-Sequenz 2269-2289
- Primer 2:: Oligonukleotid der HBV-Sequenz 2419-2439, revers

Die PCR-Ansätze werden in einem Perkin Elmer Thermal Cyler mit 30 Zyklen von 30 sec bei 92°C, 30 sec bei 50°C und 1 min bei 70°C amplifiziert.

### Beispiel 3

PCR-Amplifizierung und Markierung von HBV-DNA mit α⁻³²p dCTP.

Die Amplifikation und Markierung wird prinzipiell wie in Beispiel 2 beschrieben durchgeführt. Anstelle von dCTP werden 5 µl α⁻³² PdCTP (10 µCi/µl, 1000 nCi/nmol) eingesetzt. Die Endkonzentration von dTTP beträgt 200 µM und Dig-11-dUTP entfällt.

### Beispiel 4

### Denaturierung und Detektion amplifizierter DNA

Mit Digoxigenin markierte DNA aus einer Amplifikationsreaktion (PCR [H.A. Ehrlich ed. 1989 PCR technology: Principles and applications for DNA amplification, Stockton Press, New York], LCR (Biotechnica, EP-A-0320308), RCR (Segev Diagnostics, [WO 90/01069], oder dem System aus WO 91/03573 wird 1:10 mit Denaturierungslösung (0,05 N NaOH, 0,9 % NaCl, 0,1 % Na-Laurylsarcosin, pH 12,5) in einem Polystyrol- oder Polyethylengefäß verdünnt. Die Standzeit kann dabei zwischen 0 und 8 h variieren (Temperatur: 5°C - 45°C). 100 µl dieser Mischung werden mit 400 µl Hybridisierungspuffer, der eine biotinylierte (EP-A-0063879) Fangsonde enthält, 3 h bei 37°C in einem mit TRSA Streptavidinbeschichteten Tube (EP-A-0344578) auf einem ES 300^{R} (Boehringer Mannheim) inkubiert. Der Hybridisierungspuffer enthält 62,5 mM Na-Phosphatpuffer, 0,94 M NaCl, 94 mM Na-Citrat, 0,125 % Na-Laurylsarcosin, 0,06 % Rinderserumalbumin, pH 6,5. Die biotinylierte Fangsonde liegt mit einer Konzentration von zwischen 25 und 150 ng/ml im Hybridisierungspuffer vor. Für wie in Beispiel 2 beschrieben amplifizierte HBV-DNA wird eine biotinylierte Fangsonde der HBV_{adw}-Sequenz 2332-2351 mit 50 ng/ml eingesetzt.

Nach der Inkubation wird mit Enzymun^{R}-Waschlösung (Boehringer Mannheim) 3 x gewaschen und anschließend 500 µl Konjugatlösung (50 mU/ml Anti-Digoxigenin-POD-Konjugat (Boehringer Mannheim GmbH) in 100 mM Tris/HCl, pH 7,5, 0,9 % NaCl, 0,5 % Rinderserumalbumin) zupipettiert. Nach einer Inkubation von 30 min bei 37°C wird wiederum mit Enzymun^{R}-Waschlösung gewaschen und danach für weitere 30 min mit Substrat (ABTS^{R}, Boehringer Mannheim) inkubiert und anschließend die Extinktion bei 422 nm bestimmt.

### Beispiel 5

### Denaturierung und Detektion von nicht amplifizierter DNA

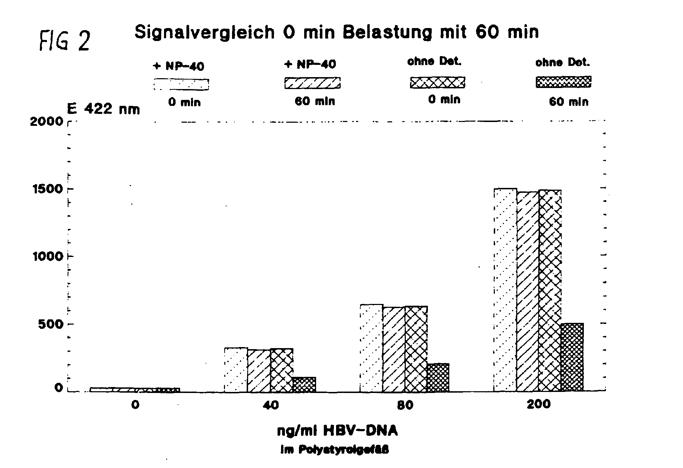

Pro Ansatz werden 10 bis 200 ng klonierte HBV-DNA (Nukleotid 29-2606 der HBV_{adw}-Sequenz kloniert in pUCBM20) mit 0,1 N NaOH, 0,9 % NaCl, 0,1 % NP-40, pH 13, in einem Polystyrolgefäß denaturiert. Zum Vergleich wird ein Versuch mit derselben Denaturierungslösung ohne NP-40 angesetzt. Nach einer Inkubationszeit von 60 min wird die denaturierte DNA mit 500 µl Hybridisierungslösung für 3 h inkubiert. Danach wird mit Enzymun^{R}-Waschlösung 3 x gewaschen und Konjugatlösung (s. Beispiel 4) zupipettiert, für 60 min bei 37°C inkubiert, wiederum gewaschen und nach der Substratreaktion (60 min) mit ABTS^{R} die Extinktion bei 422 nm gemessen. Die Ergebnisse sind FIG. 2 zu entnehmen.

Hybridisierungslösung: je 200 ng/ml biotinylierte (HBV-Sequenz 2456-2417) und mit Digoxigenin markierte (HBV-Sequenz 287-246) Oligonukleotidsonde in 62,5 mM Natriumphosphatpuffer, 0,9 M NaCl, 90 mM Na-Citrat, 0,1 % Rinderserumalbumin, 0,125 % Na-Laurylsarcosin, pH 6,5.

Die Markierung mit Digoxigenin wurde mit terminaler Transferase durchgeführt (K. Mühlegger, E. Huber, H. von der Eltz, R. Rüger & C. Kessler 1990 Biological Chemistry Hoppe-Seyler 371, 953-65).

### Beispiel 6

Vergleich verschiedener Detergenzklassen zur Stabilisierung von DNA unter alkalischen Bedingungen.

Die Detergenzkonzentration betrug 0,1 %. Benutzt wurden Polystyrolgefäße. Die weiteren Reaktionsbedingungen sind den Beispielen 1-4 zu entnehmen.

| Detergenz | NaOH mol/l | % Signal nach 240 min | Vk (0 min) | Vk (240 min) |
|---|---|---|---|---|
| ohne | 0,1 | 26,3 | 11,9 | 12,1 |
| ohne | 0,05 | 36,5 | 4,5 | 19,5 |
| | | | | |
| nichtionisch | | | | |
| | | | | |
| Synperonic | 0,05 | 98,7 | 1,2 | 0,8 |
| Tween 20 | 0,05 | 95 | 1,4 | 3,0 |
| Thesit | 0,05 | 92,7 | 1,8 | 1,3 |
| Triton X-100 | 0,05 | 95,1 | 1,2 | 1,1 |
| NP-40 | 0,05 | 99 | 0,8 | 0,8 |
| Octyl-glucopyranosid | 0,05 | 91,7 | 0,9 | 1,3 |
| | | | | |
| anionisch | | | | |
| | | | | |
| SDS (Na-Laurylsulfat) | 0,05 | 99 | 0,6 | 0,8 |
| Na-Laurylsarcosin | 0,1 | 93 | 2,9 | 1,9 |
| Na-Desoxycholat | 0,05 | 90,1 | 1,1 | 2,3 |
| | | | | |
| zwitterionisch | | | | |
| | | | | |
| Zwittergent 3-08 | 0,05 | 93,5 | 1,9 | 3,4 |
| Zwittergent 3-12 | 0,05 | 98,9 | 0,8 | 0,9 |
| Chaps | 0,1 | 93,4 | 2,2 | 2,5 |
| Chapso | 0,1 | 92,8 | 2,2 | 3,2 |
| | | | | |
| kationisch | | | | |
| | | | | |
| Cetyltrimethyl-Ammoniumbromid | 0,05 | 67 | 1,4 | 2,5 |
| Vk = Variationskoeffizient (20 Proben). | | | | |

### Beispiel 7

### Vergleich der Wirkung verschiedener Stabilisierungsregentien in unterschiedlichen Kunststoffgefäßen

| Kunststoff | Vorbehandlung | Zusatz | % Signal nach 240 min | Vk (0 min) | Vk (240 min) |
|---|---|---|---|---|---|
| PS | ohne | ohne | 36,5 | 4,5 | 19,5 |
| PS | silikonisiert | ohne | 68 | 4,4 | 12,3 |
| PS | ohne | 0,5 % R-IgG | 55 | 6,8 | 10,3 |
| PS | ohne | 100 µg/ml | 76 | 1,4 | 7,3 |
| | | Kalbsthymus-DNA | | | |
| PS | ohne | 50 µg/ml | 56,8 | 3,5 | 7,7 |
| | | Kalbsthymus-DNA | | | |
| PS | ohne | 100 µg/ml | 85,4 | 1,9 | 7,8 |
| | | Heringssp.-DNA | | | |
| PS | ohne | 100 µg/ml | 66 | 3,3 | 23,1 |
| | | Poly A DNA | | | |
| PS | ohne | 0,1 % Na-Laurylsarcosin | 95 | 2,2 | 1,1 |
| PE | ohne | ohne | 74,4 | 5,1 | 7,7 |
| PE | silikonisiert | ohne | 86,4 | 2,2 | 5,4 |
| PE | ohne | 0,1 % Na-Laurylsarcosin | 96,3 | 2,0 | 1,1 |
| Luran | ohne | ohne | 70,6 | 5,7 | 8,5 |
| Luran | ohne | 0,1 % Na-Laurylsarcosin | 94,2 | 2,3 | 1,9 |
| PS = Polystyrol; PE = Polyethylen; Luran = Copolymer aus Polystyrol und Acrylnitril Reaktionsbedindungen wie in den Beispielen 1-4. | | | | | |

## Patentansprüche

1. Verwendung eines anionischen, nicht-ionischen oder Zwitter-ionischen Detergenz zur Stabilisierung von einzelsträngigen Nukleinsäuren in alkalischen Lösungen in einem Verfahren zum Nachweis einer Nukleinsäure enthaltend folgende Schritte:
- einzelsträngig machen der Nukleinsäure unter alkalischen Bedingungen;
- Zugabe einer immobilisierbaren oder immobilisierten Fangsonde;
- Hybridisierung der Fangsonde mit der Nukleinsäure unter Immobilisierung der Nukleinsäure über die Fangsonde; und
- Nachweis der Menge an gebildetem immobilisiertem Hybrid;
**dadurch gekennzeichnet, dass** während der Behandlung unter alkalischen Bedingungen mindestens ein Detergenz aus der Gruppe der anionischen, nicht-ionischen und Zwitter-ionischen Detergentien vorhanden ist, wobei das Einzelsträngigmachen in einem Gefäß aus Polystyrol, Polyethylen oder Luran vorgenommen wird.

2. Verwendung gemäß Anspruch 1, **dadurch gekennzeichnet, dass** in allen Verfahrensschritten vom Einzelsträngigmachen an bis zur Immobilisierung mindestens ein Detergenz aus der Gruppe der anionischen, nicht-ionischen und Zwitter-ionischen Detergentien vorhanden ist.

3. Verwendung gemäß Anspruch 1, **dadurch gekennzeichnet, dass** die Konzentration des Detergenz 0,01 bis 10 Gew.-% beträgt.

4. Verwendung gemäß Anspruch 1, **dadurch gekennzeichnet, dass** die Temperatur während der Behandlung unter alkalischen Bedingungen zwischen 5 und 45 °C beträgt.

5. Verwendung gemäß Anspruch 1, **dadurch gekennzeichnet, dass** unter alkalischen Bedingungen ein pH zwischen 9,5 und 14 verstanden wird.

6. Verwendung gemäß Anspruch 1, **dadurch gekennzeichnet, dass** die alkalische Lösung mit der einzelsträngigen Nukleinsäure durch Zugabe einer Lösung der immobilisierbaren Fangsonde mit einem pH von < 7,5 neutralisiert wird.

7. Verwendung gemäß einem der vorangehenden Ansprüche, **dadurch gekennzeichnet, dass** das Verfahren vom einzelsträngig machen der Analytnukleinsäure an bis zum Nachweis der Menge des immobilisierten Hybrids auf einem Analysenautomat durchgeführt wird, welcher auch in unregelmäßigen Abständen auf die alkalisch gemachten Probenflüssigkeiten zur Detektion zugreift.

8. Verwendung eines anionischen, nicht-ionischen oder Zwitter-ionischen Detergenz zur Stabilisierung von einzelsträngigen Nukleinsäuren in alkalischen Lösungen in Gefäßen aus Polystyrol, Polyethylen oder Luran.

9. Reagenzkit zur Immobilisierung einer Nukleinsäure, enthaltend in getrennten Behältern:
- eine für eine zu immobilisierende Nukleinsäure spezifische Fangsonde,
- ein Reagenz enthaltend ein Alkalihydroxid und mindestens ein Detergenz aus der Gruppe der anionischen, nicht-ionischen und Zwitter-ionischen Detergenzien und
- ein Gefäß aus Polystyrol, Polyethylen oder Luran.

10. Reagenzkit zum Nachweis einer Nukleinsäure, enthaltend die Komponenten des Reagenzkits nach Anspruch 9, sowie mindestens eine weitere Nukleinsäure, die für die nachzuweisende Nukleinsäure spezifisch ist.

11. Reagenzkit gemäß Anspruch 10, **dadurch gekennzeichnet, dass** die weitere Nukleinsäure nachweisbar markiert ist.

12. Analysesystem zur automatischen Bestimmung von diagnostischen Parametern in Probenlösungen enthaltend:
mindestens ein Probenaufbewahrungsgefäß aus Polystyrol, Polyethylen oder Luran in einem Probenrotor,
mindestens ein Reagenzaufbewahrungsgefäß auf einem Reagenzrotor enthaltend eine Lösung zur Denaturierung von Nukleinsäuren enthaltend unter alkalischen Bedingungen mindestens ein Detergenz aus der Gruppe der anionischen, nicht-ionischen und Zwitter-ionischen Detergenzien, und
mindestens ein Reagenzaufbewahrungsgefäß enthaltend eine immobilisierbare oder immobilisierte parameterspezifische Fangsonde.

## Claims

1. Use of an anionic, non-ionic or zwitterionic detergent for stabilizing single-stranded nucleic acids in alkaline solutions in a method for detecting a nucleic acid comprising the following steps:
- making the nucleic acid single-stranded under alkaline conditions,
- adding an immobilizable or immobilized capture probe,
- hybridizing the capture probe with the nucleic acid while immobilizing the nucleic acid via the capture probe, and
- detecting the amount of immobilized hybrid that is formed
**characterized in that** at least one detergent from the group comprising anionic, non-ionic and zwitterionic detergents is present during the treatment under alkaline conditions, wherein it is made single-stranded in a vessel made of polystyrene, polyethylene or Luran.

2. Use according to claim 1, **characterized in that** a detergent from the group comprising anionic, non-ionic and zwitterionic detergents is present in all process steps from generating single strands to the immobilization.

3. Use according to claim 1, **characterized in that** the concentration of the detergent is 0.01 to 10 % by weight.

4. Use according to claim 1, **characterized in that** the temperature during the treatment under alkaline conditions is between 5 and 45°C.

5. Use according to claim 1, **characterized in that** alkaline conditions is understood as a pH between 9.5 and 14.

6. Use according to claim 1, **characterized in that** the alkaline solution containing the single-stranded nucleic acid is neutralized by adding a solution of the immobilized capture probe at a pH of < 7.5.

7. Use according to one of the previous claims, **characterized in that** the method starting from making the analyte nucleic acid single-stranded until the detection of the amount of immobilized hybrid is carried out on an automated analyzer which accesses the sample liquids made alkaline for the detection even at irregular intervals.

8. Use of an anionic, non-ionic or zwitterionic detergent to stabilize single-stranded nucleic acids in alkaline solutions in vessels made of polystyrene, polyethylene or Luran.

9. Reagent kit for immobilizing a nucleic acid containing in separate containers:
- a specific capture probe for a nucleic acid to be immobilized,
- a reagent containing an alkali hydroxide and at least one detergent from the group comprising anionic, non-ionic and zwitterionic detergents and
- a vessel made of polystyrene, polyethylene or Luran.

10. Reagent kit for detecting a nucleic acid containing the components of the reagent kit according to claim 9 as well as at least one further nucleic acid which is specific for the nucleic acid to be detected.

11. Reagent kit according to claim 10, **characterized in that** the further nucleic acid is labelled in a detectable manner.

12. Analytical system for the automatic determination of diagnostic parameters in sample solutions comprising:
at least one sample storage vessel made of polystyrene, polyethylene or Luran in a sample rotor.
at least one reagent storage vessel on a reagent rotor containing a solution for denaturing nucleic acids containing, under alkaline conditions, at least one detergent from the group comprising anionic, nori-ionic and zwitterionic detergents and at least one reagent storage vessel containing an immobilizable or immobilized parameter-specific capture probe.

## Revendications

1. Utilisation d'un détergent anionique, non ionique ou zwittérionique pour la stabilisation d'acides nucléiques monobrin dans des solutions alcalines dans un procédé de détection d'un acide nucléique, comprenant les étapes consistant :
- à transformer l'acide nucléique en une forme monobrin dans des conditions alcalines ;
- à ajouter une sonde de capture immobilisable ou immobilisée ;
- à hybrider la sonde de capture à un acide nucléique en immobilisant l'acide nucléique sur la sonde de capture;
et
- à détecter la quantité d'hybride immobilisé formé,
**caractérisée par** la présence, pendant le traitement dans des conditions alcalines, d'au moins un détergent appartenant au groupe des détergents anioniques, non ioniques et zwittérioniques, la transformation en une forme monobrin étant réalisée dans un récipient en polystyrène, polyéthylène ou Luran.

2. Utilisation selon la revendication 1, **caractérisée par** la présence, dans toutes les étapes du procédé, depuis la transformation en acide nucléique monobrin jusqu'à l'immobilisation, d'au moins un détergent appartenant au groupe des détergents anioniques, non ioniques et zwittérioniques.

3. Utilisation selon la revendication 1, **caractérisée en ce que** la concentration en détergent est de 0,01 à 10 % en poids.

4. Utilisation selon la revendication 1, **caractérisée en ce que** la température pendant le traitement dans des conditions alcalines est comprise entre 5 et 45°C.

5. Utilisation selon la revendication 1, **caractérisée en ce que** par conditions alcalines, on entend un pH compris entre 9,5 et 14.

6. Utilisation selon la revendication 1, **caractérisée en ce que** la solution alcaline contenant l'acide nucléique monobrin est neutralisée par addition d'une solution de la sonde de capture immobilisable, à pH <7,5.

7. Utilisation selon l'une quelconque des revendications précédentes, **caractérisée en ce que** le procédé consistant à transformer l'acide nucléique analyte en une forme monobrin jusqu'à la détection de la quantité d'hybride immobilisé est réalisé dans un dispositif d'analyse automatique, qui effectue également des détections à intervalles irréguliers dans l'échantillon liquide rendu alcalin.

8. Utilisation d'un détergent anionique, non ionique ou zwittérionique pour stabiliser des acides nucléiques monobrins dans des solutions alcalines dans des récipients en polystyrène, polyéthylène ou Luran.

9. Trousse de réactif destinée à immobiliser un acide nucléique, contenant, dans des récipients séparés,
- une sonde de capture spécifique d'un acide nucléique à immobiliser,
- un réactif contenant un hydroxyde alcalin et au moins un détergent appartenant au groupe des détergents anioniques, non ioniques et zwittérioniques et
- un récipient en polystyrène, en polyéthylène ou en Luran.

10. Trousse de réactif destinée à la détection d'un acide nucléique, contenant les composants de la trousse de réactif selon la revendication 9 ainsi qu'au moins un autre acide nucléique qui est spécifique pour l'acide nucléique à détecter.

11. Trousse de réactif selon la revendication 10, **caractérisée en ce que** l'acide nucléique supplémentaire est marqué de façon détectable.

12. Système d'analyse destiné à la détermination automatique de paramètres de diagnostic contenus dans des solutions d'échantillons, contenant
- au moins un récipient en polystyrène, polyéthylène ou Luran de conservation d'échantillon dans un rotor à échantillon,
- au moins un récipient de conservation de réactif dans un rotor à réactif contenant une solution destinée à la dénaturation d'acides nucléiques contenant, dans des conditions alcalines, au moins un détergent appartenant au groupe des détergents anioniques, non ioniques ou zwittérioniques, et
- au moins un récipient de conservation de réactif contenant une sonde de capture immobilisable ou immobilisée, spécifique du paramètre.
